# EUROPÄISCHE PATENTSCHRIFT

(11) **EP 1 091 806 B1**
(45) Veröffentlichungstag und Bekanntmachung des Hinweises auf die Patenterteilung: **21.08.2002**
(21) Anmeldenummer: 99927849.2
(22) Anmeldetag: 02.06.1999
(51) Int. Cl.: B01J 37/02, C07C 51/265, C07C 63/16

(54) **VERFAHREN ZUR HERSTELLUNG VON SCHALENKATALYSATOREN FÜR DIE KATALYTISCHE GASPHASENOXIDATION VON AROMATISCHEN KOHLENWASSERSTOFFEN UND SO ERHÄLTLICHE KATALYSATOREN**
METHOD FOR PRODUCING SHELL CATALYSTS FOR THE CATALYTIC VAPOR-PHASE OXIDATION OF AROMATIC HYDROCARBONS AND CATALYSTS OBTAINED IN SUCH A MANNER
PROCEDE DE PRODUCTION DE CATALYSEURS EN COQUILLE POUR L'OXYDATION CATALYTIQUE EN PHASE GAZEUSE D'HYDROCARBURES, ET CATALYSEURS OBTENUS SELON LEDIT PROCEDE

(30) Priorität: 03.06.1998 DE 19824532
(43) Veröffentlichungstag der Anmeldung: 18.04.2001
(73) Patentinhaber: BASF AKTIENGESELLSCHAFT, 67056 Ludwigshafen (DE)
(72) Erfinder: HEIDEMANN, Thomas, D-69469 Weinheim (DE); ROSOWSKI, Frank, D-68165 Mannheim (DE); LINDEN, Gerd, D-69120 Heidelberg (DE); SEUFERT, Michael, D-67098 Bad Dürkheim (DE); HEFELE, Gerhard, D-67354 Römerberg (DE); LORZ, Peter, Michael, D-67157 Wachenheim (DE)
(86) Internationale Anmeldenummer: EP9903828
(87) Internationale Veröffentlichungsnummer: WO99062637

(56) Entgegenhaltungen:
- EP-A- 0 068 192
- EP-A- 0 634 214
- EP-A- 0 807 465
- DE-A- 2 442 311

## Beschreibung

Die Erfindung betrifft ein Verfahren zur Herstellung von Schaienkatalysatoren für die katalytische Gasphasenoxidation von aromatischen Kohlenwasserstoffen zu Carbonsäuren und/oder Carbonsäureanhydriden, auf deren Trägermaterial unter Verwendung bestimmter Bindemittel eine Schicht aus katalytisch aktiven Metalloxiden schalenförmig aufgebracht ist, so erhältliche Katalysatoren, sowie ein Verfahren für die katalytische Gasphasen oxidation von aromatischen Kohlenwasserstoffen zu Carbonsäuren und/oder Carbonsäureanhydriden mit einem molekularen Sauerstoff enthaltenden Gas in einem Festbett unter Verwendung dieser Katalysatoren.

Bekanntermaßen wird eine Vielzahl von Carbonsäuren und/oder Carbonsäureanhydriden technisch durch die katalytische Gasphasenoxidation von aromatischen Kohlenwasserstoffen, wie Benzol, den Xylolen, Naphthalin, Toluol oder Durol in Festbettreaktoren, vorzugsweise Rohrbündelreaktoren, hergestellt. Auf diese Weise werden beispielsweise Benzoesäure, Maleinsäureanhydrid, Phthalsäureanhydrid, Isophthalsäure, Terephthalsäure oder Pyromellithsäureanhydrid gewonnen. Dazu wird im allgemeinen ein Gemisch aus einem molekularen Sauerstoff enthaltenden Gas, beispielsweise Luft und das zu oxidierende Ausgangsmaterial durch eine Vielzahl in einem Reaktor angeordneter Rohre geleitet, in denen sich eine Schüttung mindestens eines Katalysators befindet. Zur Temperaturregelung sind die Rohre von einem Wärmeträgermedium, beispielsweise einer Salzschmelze, umgeben. Trotz dieser Thermostatisierung kann es in der Katalysatorschüttung zur Ausbildung sogenannter "Heißer Flecken" (hot spots) kommen, in denen eine höhere Temperatur herrscht als im übrigen Teil der Katalysatorschüttung. Diese "hot spots" geben Anlaß zu Nebenreaktionen, wie der Totalverbrennung des Ausgangsmaterials oder führen zur Bildung unerwünschter, vom Reaktionsprodukt nicht oder nur mit viel Aufwand abtrennbarer Nebenprodukte, beispielsweise zur Bildung von Phthalid oder Benzoesäure, bei der Herstellung von Phthalsäureanhydrid (PSA) aus o-Xylol. Des weiteren verhindert die Ausbildung eines ausgeprägten hot spots ein schnelles Anfahren des Reaktors da ab einer bestimmten hot spot-Temperatur der Katalysator irreversibel geschädigt werden kann, so daß die Beladungserhöhung nur in kleinen Schritten durchführbar ist und sehr sorgfältig kontrolliert werden muß.

Zur Abschwächung dieses hot spots wurde in der Technik dazu übergegangen, unterschiedlich aktive Katalysatoren schichtweise in der Katalysatorschüttung anzuordnen, wobei in der Regel der weniger aktive Katalysator so im Festbett angeordnet ist, daß das Reaktionsgasgemisch mit ihm als erstes in Kontakt kommt, d.h. er liegt in der Schüttung zum Gaseintritt hin, wohingegen der aktivere Katalysator zum Gasaustritt aus der Katalysatorschüttung hin gelegen ist. So können entweder die unterschiedlich aktiven Katalysatoren in der Katalysatorschüttung bei der gleichen Temperatur dem Reaktionsgas ausgesetzt werden, oder es können die beiden Schichten aus unterschiedlich aktiven Katalysatoren aber auch auf unterschiedliche Reaktionstemperaturen thermostatisiert mit dem Reaktionsgas in Kontakt gebracht werden, wie dies in DE-A 40 130 51 beschrieben ist.

Als Katalysatoren haben sich sogenannte Schalenkatalysatoren bewährt, bei denen die katalytisch aktive Masse schalenförmig auf einem Kern von im allgemeinen unter den Reaktionsbedingungen inerten Trägermaterial wie Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien aufgebracht ist. Als katalytisch aktiver Bestandteil der katalytisch aktiven Masse dieser Schalenkatalysatoren dient im allgemeinen neben Titandioxid in Form seiner Anatasmodifikation Vanadiumpentoxid. Des weiteren können in der katalytisch aktiven Masse in geringen Mengen eine Vielzahl anderer oxidischer Verbindungen enthalten sein, die als Promotoren die Aktivität und Selektiviät des Katalysators beeinflussen, beispielsweise in dem sie seine Aktivität absenken oder erhöhen. Als solche Promotoren seien beispielhaft die Alkalimetalloxide, insbesondere Lithium-, Kalium-, Rubidiumund Cäsiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid, und Phosphorpentoxid genannt. Als die Aktivität vermindernder und die Selektivität erhöhender Promotor wirken z.B. die Alkalimetalloxide, wohingegen oxidische Phosphorverbindungen, insbesondere Phosphorpentoxid die Aktivität des Katalysators erhöhen, aber dessen Selektivität vermindern. Diese Bestandteile sind sämtlich aus der einschlägigen Fachliteratur bekannt. Beispielsweise wird auf die Zusammenfassende Darstellung in WO 98/00778 verwiesen.

Zur Herstellung derartiger Schalenkatalysatoren wird z.B. nach den Verfahren von DE-A 16 42 938 und DE-A 17 69 998 eine wäßrige und/oder ein organisches Lösungsmittel enthaltende Lösung oder Suspension der Aktivmassenbestandteile und/oder deren Vorläuferverbindungen, welche im folgenden als "Maische" bezeichnet wird, auf das Trägermaterial in einer beheizten Dragiertrommel bei erhöhter Temperatur aufgesprüht, bis der gewünschte Aktivmassenanteil am Katalysatorgesamtgewicht erreicht ist. Nach DE 21 06 796 läßt sich die Beschichtung auch in Wirbelbeschichtern durchführen, wie sie z.B. in der DE 12 80 756 beschrieben sind. Beim Aufsprühen in der Dragiertrommel sowie bei Beschichten im Wirbelbett treten allerdings hohe Verluste auf, da erhebliche Mengen der Maische vernebelt bzw. durch Abrasion Teile der bereits aufgeschichteten Aktivmasse wieder abgerieben und durch das Abgas ausgetragen werden. Da der Aktivmasseanteil am Gesamtkatalysator im allgemeinen nur eine geringe Abweichung vom Sollwert haben soll, da durch die Menge der aufgebrachten Aktivmasse und die Schichtdicke der Schale Aktivität und Selektivität des Katalysators stark beeinflußt werden, muß der Katalysator bei den geschilderten Herstellungsweisen häufig zur Bestimmung der aufgebrachten Aktivmassenmenge abgekühlt, aus der Dragiertrommel bzw. der Wirbelschicht entnommen und nachgewogen werden. Wird zuviel Aktivmasse auf dem Katalysatorträger abgeschieden, ist im allgemeinen eine nachträgliche, schonende Entfernung der zu viel aufgetragenen Aktivmassenmenge ohne Beeinträchtigung der Festigkeit der Schale, insbesondere ohne Rißbildung in der Katalysatorschale, nicht möglich.

Um diese Probleme abzumindern, wurde in der Technik dazu übergegangen, der Maische organische Binder, bevorzugt Copolymere, vorteilhaft in Form einer wäßrigen Dispersion, von Vinylacetat/Vinyllaurat, Vinylacetat/Acrylat, Vinylacetat/Maleat sowie Vinylacetat/Ethylen zuzusetzen, wobei gemäß EP-A 07 442 14 Bindermengen von 10-20 Gew.-%, bezogen auf den Feststoffgehalt der Maische, eingesetzt wurden. Wird die Maische ohne organische Bindemittel auf den Träger aufgetragen, so sind Beschichtungstemperaturen über 150°C von Vorteil. Bei Zusatz der angegebener Bindemittel liegen gemäß DE 210 67 96 die brauchbaren Beschichtungstemperaturen mit 70-130°C deutlich niedriger. Die aufgetragenen Bindemittel brennen nach dem Einfüllen des Katalysators und Inbetriebnahme des Reaktors innerhalb kurzer Zeit aus. Der Binderzusatz hat zudem den Vorteil, daß die Aktivmasse gut auf dem Träger haftet, so daß Transport und Einfüllen des Katalysators erleichtert werden.

Bei dem Abbrand kommt es allerdings zu einer Lockerung der Haftung der Aktivmassenschicht auf dem Träger. Dies ist normalerweise nicht kritisch, da der Katalysator im Reaktorrohr keinen starken mechanischen Beanspruchungen mehr ausgesetzt ist. Insbesondere bei größeren Mengen an Binderzusatz ist aber nicht auszuschließen, daß die Aktivmassenschicht derart gelockert wird, daß sie unter Reakticnsbedingungen durch das durchströmende Gasgemisch langsam abgetragen wird. Dies hat zur Folge, daß die Langzeitstabilität des Katalysators verringert wird und die für die notwendige PSA-Qualität einzustellende Salzbadtemperatur des Reaktors zügig erhöht werden muß, was sich wiederum negativ auf die erzielbare PSA-Ausbeute auswirkt. Desweiteren kann es bei Abbrand des Binderzusatzes neben Geruchsbelästigungen und weiteren Umweltverträglichkeitsbeeinträchtigungen durch Entzünden von Zersetzungsprodukten des Binderzusatzes zu Verpuffungen kommen, die den sicheren Betrieb des Reaktors gefährden können. Schließlich ist es aus Kostengründen sinnvoll, die für den gewünschten Effekt des Binderzusatzes notwendige Menge zu minimieren.

Der vorliegenden Erfindung lag somit die Aufgabe zugrunde, ein Verfahren zur Herstellung von Schalenkatalysatoren für die katalytische Gasphasenoxidation von aromatischen Kohlenwasserstoffen zu Carbonsäuren und/oder Carbonsäureanhydriden zu finden, das unter Beibehaltung der durch den Binderzusatz erzielbaren Vorteile die geschilderten Nachteile des Binderzusatzes vermeidet.

Die Aufgabe der Erfindung wird, kurz gefaßt, durch Zusatz eines speziellen organischen Bindemittels gelöst, das überraschenderweise bereits in Zusätzen <10Gew.-%, bezogen auf den Feststoffgehalt der Maische, zu den oben genannten gewünschten Vorteilen führt. Im einzelnen wurde die Aufgabe erfindungsgemäß gelöst mit einem Verfahren zur Herstellung von Schalenkatalysatoren für die katalytische Gasphasenoxidation von aromatischen Carbonsäuren und/oder Carbonsäureanhydriden, bestehend aus einem Trägerkern und darauf schalenförmig aufgebrachten katalytisch wirksamen Metalloxiden, erhalten durch Aufsprühen einer die aktiven Metalloxider enthaltenden wässrigen Aktivmassensuspension bei höheren Temperaturen auf das 50 bis 450°C heiße Trägermaterial, dadurch gekennzeichnet, daß die wässrige Aktivmassensuspension 1 bis 10 Gew.-%, bezogen auf den Feststoffgehalt der Aktivmassensuspension, eines Bindemittels enthält, bestehend aus
A) einem durch radikalische Polymerisation erhaltenem Polymerisat, enthaltend 5 bis 100 Gew.-% Monomere (a) in Form von ethylenisch ungesattigten Säureanhydriden oder ethylenisch ungesättigten Dicarbonsäuren, deren Carboxylgruppen ein Anhydrid bilden können und 0 bis 95 Gew.-% weiterer monoethylensch ungesättigter Monomere(b), mit der Maßgabe, daß die Monomeren (a) und (b) durchschnittlich höchstens 5, vorzugsweise 2 bis 4 Kohlenstoffatome die nicht durch Sauerstoff enthaltende Gruppen funktionalisiert sind, aufweisen und
B) einem Alkanolamin mit mindestens 2 OH-Gruppen, höchstens 2 Stickstoffatomen, vorzugsweise einem Stickstoffatom und höchstens 8 C-Atomen,
wobei das Gewichtsverhältnis A:B 1:0,05 bis 1:1 beträgt.

Das Alkandamin hat dabei die Funktion eines über die Bildung von Estergruppen als Vernetzer wirkenden Agens. Diese "Härtung" tritt beim Aufsprühen der "Maische" auf den Träger bei erhöhter Temperatur ein.

Die erfindungsgemäß zu verwendenden Bindemittel sollen möglichst wenige Kohlenstoffatome in der Kette, also möglichst wenige nicht Sauerstoff enthaltende Gruppen, wie nicht durch OH, COOH oder COOR Gruppen funktionalisierte C-Atome enthalten, um die Wärmeentwicklung beim späteren Abbrennen des Bindemittels im Reaktor gering zu halten und eine Schädigung durch Überhitzen des Katalysators zu vermeiden. Diese Bedingungen wird durch die oben angegebene Charakterisierung der Gesamtkohlenstoffzahl des Monomeren ausgedrückt.

Nach einer anderen Definition, die den gleichen Sachverhalt charakterisiert, beträgt das Atomverhältnis C:O im Bindemittel höchstens 3:1, vorzugsweise bis 2,5:1 und besonders bevorzugt bis 2:1.

Im einzelnen kommen für das erfindungsgemäße Verfahren die in WO 97/31036 beschriebenen Bindemittel in Betracht, die die vorgenannten Bedingungen erfüllen. Im Hinblick auf die angegebenen Auswahlkriterien wird deshalb auf die Angaben bezüglich der Bindemittel auf die WO 97/31036 ausdrücklich verwiesen.

Demgemäß werden als Monomere (a) bevorzugt Maleinsäure, Maleinsäureanhydrid, Itaconsäure, 1,2,3,6-Tetrahydrophtalsäure, 1,2,3,6 -Tetrahydrophthalsäureanhydrid, deren Alkali- und Ammoniumsalze oder Mischungen daraus eingesetzt. Besonders bevorzugt sind Maleinsäure und Maleinsäureanhydrid.

Als Monomere (b) können beispielsweise eingesetzt werden: Monoethylenisch ungesättigte C₃-bis C₆-Monocarbonsauren, wie z.B. Acrylsäure, Methacrylsäure, Ethylacrylsäure, Allylessigsäure, Crotonsäure, Vinylessigsäure, Maleinsäurehalbester wie Maleinsäuremonomethylester, deren Mischungen bzw. deren Alkali- und Ammoniumsalze, ferner
Vinyl- und Allylalkylether, wobei der Alkylrest noch weitere Substituenten wie eine Hydroxylgruppe, eine bzw. mehrere Alkoxylatgruppen tragen kann, wie z.B. Methylvinylether, Ethylvinylether, Propylvinylether, Isobutylvinylether, Vinyl-4-hydroxybutylether, sowie die entsprechenden Allylether bzw. deren Mischungen,
Arcylamide und alkylsubstituierte Acrylamide , wie z.B. Acrylamid, Methacrylamid, N-tert.-Butylacrylamid, N-Methyl (meth)acrylamid,
Sulfogruppenhaltige Monomere, wie z.B. Allylsulfonsäure, Methallylsulfonsäure, Styrolsulfonat, Vinylsulfonsäure, Allyloxybenzolsulfonsäure, 2-Acrylamido-2-methylpropansulfonsäure, deren entsprechende Alkali- oder Ammoniumsalze bzw. deren Mischungen,
C₁-bis C₄-Alkylester oder C₁- bis C₄-Hydroxyalkylester der Acrylsäure, Methacrylsäure oder Maleinsäure oder Ester von mit 2 bis 50 Mol Ethylenoxid, Propylenoxid, Butylenoxid oder Mischungen davon alkoxylierten C₁-bis C₄-Alkoholen mit Acrylsäure, Methacrylsäure oder Maleinsäure (Monomere b₆), wie z.B. Methyl(meth)acrylat oder Ethyl(meth)acrylat,
Vinyl- und Allylester von C₁-bis C₄-Monocarbonsäuren wie z.B. Vinylformiat, Vinylacetat, Vinylpropionat oder Vinylbutyrat. Als weitere Monomere seien noch genannt: Alkylalkohol, Acrolein, Methacrolein bzw. Mischungen davon.

Bevorzugte Monomere (b) sind Acrylsäure, Methacrylsäure, Methylvinylether, Ethylvinylether, Vinylacetat bzw. Mischungen davon.

Besonders bevorzugt sind Acrylsäure, Methacrylsäure, bzw. Mischungen davon.

Die Monomere (b) sind im Polymerisat bevorzugt mit 20 bis 90 Gew.-%, insbesondere mit 50 bis 80 Gew.-% enthalten.

Die Polymerisate aus den Monomeren (a) und gegebenenfalls (b) können nach üblichen Polymerisationsverfahren hergestellt werden, z.E. durch Substanz-, Emulsions-, Suspensions-, Dispersions-, Fällungs- und Lösungspolymerisation. Bei den genannten Polymerisationsverfahren wird bevorzugt unter Ausschluß von Sauerstoff gearbeitet, vorzugsweise in einem Stickstoffstrom. Für alle Polymerisationsmethoden werden die üblichen Apparaturen verwendet, z.B. Rührkessel, Rührkesselkaskaden, Autoklaven, Rohrreaktoren und Kneter. Bevorzugt wird nach der Methode der Lösungs-, Emulsions-, Fällungs- oder Suspensionspolymerisation gearbeitet. Besonders bevorzugt sind die Methoden der Lösungs- und Emulsionspolymerisation. Die Polymerisation kann in Lösungs- oder Verdünnungsmitteln, wie z.B. Toluol, o-Xylol, p-Xylol, Cumol, Chlorbenzol, Ethylbenzol, technischen Mischungen von Alkylaromaten, Cyclohexan, technischen Aliphatenmischungen, Aceton, Cyclohexanon, Tetrahydrofuran, Dioxan, Glykolen und Glykolderivaten, Polyalkylenglykolen und deren Derivate, Diethylether, tert.-Butylmethylether, Essigsäuremethylester, Isopropanol, Ethanol, Wasser oder Mischungen wie z.B. Isopropanol/Wasser-Mischungen ausgeführt werden. Vorzugsweise wird als Lösungs- oder Verdünnungsmittel Wasser, gegebenenfalls mit Anteilen bis zu 60 Gew.-% an Alkoholen oder Glykolen verwendet. Besonders bevorzugt wird Wasser eingesetzt.

Die Polymerisation kann bei Temperaturen von 20 bis 300, vorzugsweise von 60 bis 200°C durchgeführt werden. Je nach Wahl der Polymerisationsbedingungen lassen sich gewichtsmittlere Molekulargewichte z.B. von 800 bis 5 000 000, insbesondere von 1 000 bis 1 000 000 einstellen. Bevorzugt liegen die gewichtsmittleren Molekulargewichte M_{w} über 15.000. Besonders bevorzugt sind gewichtsmittlere Molekulargewichte von 15 000 bis 600 000. M_{w} wird bestimmt durch Gelpermeationschromatographie.

Die Polymerisation wird vorzugsweise in Gegenwart von Radikale bildenden Verbindungen durchgeführt. Man benötigt von diesen Verbindungen bis zu 30, vorzugsweise 0,05 bis 15, besonders bevorzugt 0,2 bis 8 Gew.-%, bezogen auf die bei der Polymerisation eingesetzten Monomeren. Bei mehrkomponentigen Initiatorsystemen (z.B. Redox-Initiatorsystemen) beziehen sich die vorstehenden Gewichtsangaben auf die Summe der Komponenten.

Geeignete Polymerisationsinitiatoren sind beispielsweise Peroxide, Hydroperoxide, Peroxidisulfate, Percarbonate, Peroxiester, Wasserstoffperoxid und Azoverbindungen. Beispiele für Initiatoren, die wasserlöslich oder auch wasserunlöslich sein können, sind Wasserstoffperoxid, Dibenzoylperoxid, Dicyclohexylperoxidicarbonat, Dilauroylperoxid, Methylethylketonperoxid, Di-tert.-Butylperoxid, Acetylacetonperoxid, tert.-Butylhydroperoxid, Cumolhydroperoxid, tert.-Butylperneodecanoat, tert.-Amylperpivalat, tert.-Butylperpivalat, cert.-Butylperneohexanoac, tert.-Butylper-2-ethylhexanoat, tert.-Butyl-perbenzoat, Lithium-, Natrium-, Kalium- und Ammoniumperoxidisulfat, Azodiisobutyronitril, 2,2'-Azobis(2-amidinopropan)dihydrochlorid, 2-(Carbamoylazo)isobutyronitril und 4,4-Azobis(4-cyanovaleriansäure).

Die Initiatoren können allein oder in Mischung untereinander angewendet werden, z.B. Mischungen aus Wasserstoffperoxid und Natriumperoxidisulfat. Für die Polymerisation in wäßrigem Medium werden bevorzugt wasserlösliche Initiatoren eingesetzt.

Auch die bekannten Redox-Initiatorsysteme können als Polymerisationsinitiatoren verwendet werden. Solche Redox-Initiatorsysteme enthalten mindestens eine peroxidhaltige Verbindung in Kombination mit einem Redox-Coinitiator z.B. reduzierend wirkenden Schwefelverbindungen, beispielsweise Bisulfite, Sulfite, Thiosulfate, Dithionite und Tetrathionate von Alkalimetallen und Ammoniumverbindungen. So kann man Kombinationen von Peroxodisulfaten mit Alkalimetall- oder Ammoniumhydrogensulfiten einsetzen, z.B. Ammoniumperoxidisulfat und Ammoniumdisulfit. Die Menge der peroxidhaltigen Verbindung zum Redox-Coinitiator beträgt 30:1 bis 0,05:1.

In Kombination mit den Initiatoren bzw. den Redoxinitiatorsystemen können zusätzlich Übergangsmetallkatalysatoren eingesetzt werden, z.B. Salze von Eisen, Kobalt, Nickel, Kupfer, Vanadium und Mangan. Geeignete Salz sind z.B. Eisen-II-sulfat, Kobalt-II-chlorid, Nickel-II-sulfat, Kupfer-I-chlorid. Bezogen auf Monomeren wird das reduzierend wirkende Übergangsmetallsalz in einer Konzentration von 0,1 ppm bis 1 000 ppm eingesetzt. So kann man Kombinationen von Wasserstoffperoxid mit Eisen-II-Salzen einsetzen, wie beispielsweise 0,5 bis 30 % Wasserstoffperoxid und 0,1 bis 500 ppm Mohrsches Salz.

Auch bei der Polymerisation in organischen Lösungsmitteln können in Kombination mit den obengenannten Initiatoren Redox-Coinitiatoren und/oder Übergangsmetallkatalysatoren mitverwendet werden, z.B. Benzoin, Dimethylanilin, Ascorbinsäure sowie organisch lösliche Komplexe von Schwermetallen, wie Kupfer, Cobalt, Eisen, Mangan, Nickel und Chrom. Die üblicherweise verwendeten Mengen an Redox-Coinitiatoren bzw. Übergangsmetallkatalysatoren betragen hier üblicherweise etwa 0,1 bis 1 000 ppm, bezogen auf die eingesetzten Mengen an Monomeren.

Falls die Reaktionsmischung an der unteren Grenze des für die Polymerisation in Betracht kommenden Temperaturbereiches anpolymerisiert und anschließend bei einer höheren Temperatur auspolymerisiert wird, ist es zweckmäßig, mindestens zwei verschiedene Initiatoren zu verwenden, die bei unterschiedlichen Temperaturen zerfallen, so daß in jedem Temperaturintervall eine ausreichende Konzentration an Radikalen zur Verfügung steht.

Um Polymerisate mit niedrigem mittleren Molekulargewicht herzustellen, ist es oft zweckmäßig, die Copolymerisation in Gegenwart von Reglern durchzuführen. Hierfür können übliche Regler verwendet werden, wie beispielsweise organische SH-Gruppen enthaltende Verbindungen, wie 2-Mercaptoethanol, 2-Mercaptopropanol, Mercaptoessigsäure, tert.-Butylmercaptan, n-Octylmercaptan, n-Dodecylmercaptan und tert.-Dodecylmercaptan, C₁- bis C₄-Aldehyde, wie Formaldehyd, Acetaldehyd, Propionaldehyd, Hydroxylammoniumsalze wie Hydroxylammoniumsulfat, Ameisensäure, Natriumbisulfit oder Isopropanol. Die Polymerisationsregler werden im allgemeinen in Mengen von 0,1 bis 10 Gew.-%, bezogen auf die Monomeren eingesetzt. Auch durch die Wahl des geeigneten Lösungsmittels kann auf das mittlere Molekulargewicht Einfluß genommen werden. So führt die Polymerisation in Gegenwart von Verdünnungsmitteln mit benzylischen H-Atomen zu einer Verringerung des mittleren Molekulargewichtes durch Kettenübertragung.

Wird nach der Methode der Emulsions-, Fällungs-, Suspensionsoder Dispersionspolymerisation gearbeitet, so kann es vorteilhaft sein, die Polymertröpfchen bzw. Polymerteilchen durch grenzflächenaktive Hilfsstoffe zu stabilisieren. Typischerweise verwendet man hierzu Emulgatoren oder Schutzkolloide. Es kommen anionische, nichtionische, kationische und amphotere Emulgatoren in Betracht. Anionische Emulgatoren sind beispielsweise Alkylbenzolsulfonsäuren, sulfonierte Fettsäuren, Sulfosuccinate, Fettalkoholsulfate, Alkylphenolsulfate und Fettalkoholethersulfate. Als nichtionische Emulgatoren können beispielsweise Alkylphenolethoxylate, Primäralkoholethoxilate, Fettsäureethoxilate, Alkanolamidethoxilate, Fettaminethoxilate, EO/PO-Blockcopolymere und Alkylpolyglucoside verwendet werden. Als kationische bzw. amphotere Emulgatoren werden beispielsweise verwendet: Quaternisierte Aminalkoxylate, Alkylbetaine, Alkylamidobetaine und Sulfobetaine.

Typische Schutzkolloide sind beispielsweise Cellulosederivate, Polyethylenglykol, Polypropylenglykol, Copolymerisate aus Ethylenglykol und Propylenglykol, Polyvinylacetat, Polyvinylalkohol, Polyvinylether, Stärke und Stärkederivate, Dextran, Polyvinylpyrrolidon, Polyvinylpyridin, Polyethylenimin, Polyvinylimidazol, Polyvinylsuccinimid, Polyvinyl-2-methylsuccinimid, Polyvinyl-1,3-oxazolidon-2, Polyvinyl-2-methylimidazolin und Maleinsäure bzw. Maleinsäureanhydrid enthaltende Copolymerisate, wie sie z.B. in DE 2 501 123 beschrieben sind.

Die Emulgatoren oder Schutzkolloide werden üblicherweise in Konzentrationen von 0,05 bis 20 Gew.-%, bezogen auf die Monomere, eingesetzt.

Wird in wäßriger Lösung oder Verdünnung polymerisiert, so können die Monomere vor oder während der Polymerisation ganz oder teilweise durch Basen neutralisiert werden. Als Basen kommen vorzugsweise stickstofffreie Basen, beispielsweise Alkali- oder Erdalkaliverbindungen wie Natriumhydroxid, Kaliumhydroxid, Calciumhydroxid, Magnesiumoxid, Natriumcarbonat in Betracht.

Besonders bevorzugt werden die ethylenisch ungesättigten Carbonsäuren vor und während der Polymerisation nicht neutralisiert. Bevorzugt wird auch nach der Polymerisation kein Neutralisierungsmittel, abgesehen vom Alkanolamin (B), zugesetzt. Die Durchführung der Polymerisation kann nach einer Vielzahl von Varianten kontinuierlich oder diskontinuierlich durchgeführt werden. Üblicherweise legt man einen Teil der Monomeren gegebenenfalls in einem geeigneten Verdünnungsmittel oder Lösungsmittel und gegebenenfalls in Anwesenheit eines Emulgators, eines Schutzkolloids oder weiterer Hilfsstoffe vor, inertisiert, und erhöht die Temperatur bis zum Erreichen der gewünschten Polymerisationstemperatur. Es kann allerdings auch lediglich ein geeignetes Verdünnungsmittel vorgelegt sein. Innerhalb eines definierten Zeitraumes werden der Radikalinitiator, weitere Monomere und sonstige Hilfsstoffe, wie z.B. Regler oder Vernetzer jeweils gegebenenfalls in einem Verdünnungsmittel zudosiert. Die Zulaufzeiten können unterschiedlich lang gewählt werden. Beispielsweise kann man für den Initiatorzulauf eine längere Zulaufzeit wählen als für den Monomerzulauf.

Wird das Polymerisat nach dem Verfahren einer Lösungspolymerisation in Wasser gewonnen, so ist üblicherweise keine Abtrennung des Lösungsmittels notwendig. Besteht dennoch der Wunsch, das Polymerisat zu isolieren, kann z.B. eine Sprühtrocknung durchgeführt werden.

Wird das Polymerisat nach der Methode einer Lösungs-, Fällungsoder Suspensionspolymerisation in einem wasserdampfflüchtigen Lösungsmittel oder Lösungsmittelgemisch hergestellt, so kann das Lösungsmittel durch Einleiten von Wasserdampf abgetrennt werden, um so zu einer wäßrigen Lösung oder Dispersion zu gelangen. Das Polymerisat kann von dem organischen Verdünnungsmittel auch durch einen Trocknungsprozeß abgetrennt werden.

Bevorzugt liegen die Polymerisate (A) in Form einer wäßrigen Dispersion oder Lösung mit Feststoffgehalten von vorzugsweise 10 bis 80 Gew.-%, insbesondere 40 bis 65 Gew.-% vor.

Polymerisat (A) kann auch durch Pfropfung von Maleinsäure bzw. Maleinsäureanhydrid bzw. einer Maleinsäure oder Maleinsäureanhydrid enthaltenden Monomermischung auf eine Pfropfgrundlage erhalten werden. Geeignete Pfropfgrundlagen sind beispielsweise Monosaccharide, Oligosaccharide, modifizierte Polysaccharide und Alkylpolyglykolether. Solche Pfropfpolymerisate sind beispielsweise in DE 4 003 172 und EP 116 930 beschrieben.

Als Komponente B) werden Alkanolamine mit mindestens zwei OH-Gruppen und höchstens 2 Stickstoffatomen, vorzugsweise mit einem Stickstoffatom eingesetzt. Bevorzugt sind Alkanolamine der Formel 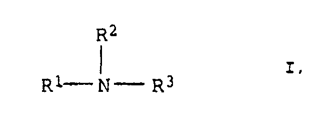 in der R¹ für ein H-Atom, eine C₁-C₄-Alkylgruppe oder eine C₁-C₄-Hydroxyalkylgruppe steht und R² und R³ für eine C₁-C₄-Hydroxyalkylgruppe stehen.

Besonders bevorzugt stehen R² und R³ unabhängig voneinander für eine C₂-C₄-Hydroxyalkylgruppe und R¹ für ein H-Atom, eine C₁-C₃-Alkylgruppe oder eine C₂-C₄-Hydroxyalkylgruppe.

Als Verbindungen der Formel I seien z.B. Diethanolamin, Triethanolamin, Diisopropanolamin, Triisopropanolamin, Methyldiethanolamin, Butyldiethanolamin und Methyldiisopropanolamin genannt. Besonders bevorzugt ist Triethanolamin.

Zur Herstellung der erfindungsgemäßen zu verwendenden Bindemittel werden das Polymerisat (A) und das Alkanolamin (B) bevorzugt in einem solchen Verhältnis zueinander eingesetzt, daß das Molverhältnis von Carboxylgruppen der Komponente (A) und der Hydroxylgruppen der Komponente (B) 20:1 bis 1:1, bevorzugt 8:1 bis 5:1 und besonders bevorzugt 5:1 bis 1,7:1 beträgt (die Anhydridgruppen werden hierbei als 2 Carboxylgruppen gerechnet).

Die Herstellung der Bindemittel erfolgt dabei z.B. einfach durch Zugabe des Alkanolamins zur wäßrigen Dispersion oder Lösung der Polymerisate (A).

Die erfindungsgemäßen zu verwendenden Bindemittel enthalten vorzugsweise weniger als 1,0 Gew.-%, besonders bevorzugt weniger als 0,5 Gew.-% und ganz besonders bevorzugt weniger als 0,3 Gew.-%, insbesondere weniger als 0,1 Gew.-%, bezogen auf die Summe aus (A) + (B) eines Phosphor enthaltenden Reaktionsbeschleunigers. Phosphor enthaltende Reaktionsbeschleuniger sind in EP-A 651 088 und EP-A 583 086 genannt. Es handelt sich dabei insbesondere um Alkalimetallhypophoshpite, -phosphite, -polyphosphate, -dihydrogenphosphate, Polyphosphorsäure, Hypophosphorsäure, Phosphorsäure, Alkylphosphinsäure oder Oligomere bzw. Polymere dieser Salze und Säuren.

Die Bindemittel enthalten aber vorzugsweise keine Phosphor enthaltenden Reaktionsbeschleuniger bzw. keine zur Reaktionsbeschleunigung wirksame Mengen einer Phosphor enthaltenden Verbindung. Die Bindemittel können einen Veresterungskatalysator enthalten, wie z.B. Schwefelsäure oder p-Toluolsulfonsäure. Die erfindungsgemäß zu verwendenden Bindemittel werden in der Regel als alleinige Bindemittel für die Herstellung der Schalenkatalysatoren verwendet. Sie können jedoch auch zusammen mit anderen Bindemitteln angewandt werden.

Die nach diesem Verfahren unter Zusatz des erfindungsgemäß zu verwendenden Bindemittels hergestellten Katalysatoren sind prinzipiell keine anderen als die in den eingangs genannten Patentschriften beschriebenen, auf die hiermit Bezug genommen wird. Dies sind in der Regel sogenannte Schalenkatalysatoren, bei denen die katalytisch aktive Masse schalenförmig auf einem im allgemeinen unter den Reaktionsbedingungen inerten Trägermaterial, wie Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien aufgebracht ist. Als katalytisch aktiver Bestandteil der katalytisch aktiven Masse dieser Schalenkatalysatoren dient im allgemeinen neben Titandioxid in form seiner Anatasmodifikation Vanadiumpentoxid. Des weiteren können in der katalytisch aktiven Masse in geringen Mengen eine Vielzahl anderer oxidscher Verbindungen enthalten sein, die als Promotoren die Aktivität und Selektivität des Katalysators beeinflussen, beispielsweise in dem sie seine Aktivität absenken oder erhöhen. Als solche Promotoren seine beispielhaft die Alkalimetalloxide, insbesondere Lithium-, Kalium-, Rubidium- und Cäsiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid genannt. Als die Aktivität vermindernder und die Selektivität erhöhender Promotor wirken z.B. die Alkalimetalloxide, wohingegen oxidische Phosphorverbindungen, insbesondere Phosphorpentoxid die Aktivität des Katalysators erhöhen, aber dessen Selektivität vermindern.

Die Herstellung der Katalysatoren erfolgt durch Aufbringen der aktiven Masse auf den Träger in an sich üblicher Weise durch Aufsprühen einer Aktivmassenmaische auf einen auf 50-450°C vorerhitzten Träger z.B. in einer Dragiertrommel oder in Wirbelbettbeschichtern wie dies z.B. in DE 21 06 796, DE 17 69 998, DE 16 42 938 oder DE 25 10 994 beschrieben ist. Vor Aufsprühbeginn wird der Maische das Bindemittel zugesetzt. Bevorzugt wird eine Beschichtung im Temperaturbereich 100 bis 250°C und besonders bevorzugt bei 140 bis 200°C durchgeführt, da in diesen Temperaturbereichen eine optimale Aushärtung des Binderzusatzes erfolgt.

Dieser Aushärtvorgang während der Beschichtung hat zur Folge, daß bereits geringe Zusätze von 1 bis 10 Gew.-%, bevorzugt 4 bis 8 Gew.-% Bindemittel, bezogen auf den Feststoffgehalt der Maische, ausreichen, um die Haftfestigkeit der Aktivmasse auf dem Träger deutlich zu erhöhen und eine deutliche Verringerung der Maischeverluste beim Beschichtungsvorgang zu bewirken. Eine Erhöhung des Binderzusatzes auf über 10 Gew.-% führt zwar noch zu einer weiteren geringfügigen unmittelbaren Verbesserung der Haftfestigkeit; diese größeren Mengen an Binderzusatz führen aber auch dazu, daß die Haftfestigkeit der aktiven Masse nach Calcination des Katalysators bei 400°C stark erniedrigt wird, während Katalysatoren mit Binderzusätzen von weniger als 10 Gew.-% auch nach Calcination noch gute Abriebwerte aufweisen.

Desweiteren sind beim Abbrand der erforderlichen geringen Mengen des neuartigen Binderzusatzes nach Einfüllen des Katalysators in den Reaktor und Inbetriebnahme des Reaktors weder Geruchsbelästigungen noch weitere Umweltverträglichkeitsbeeinträchtigungen festzustellen. Entzündungen von Zersetzungsprodukten des Binderzusatzes, die zu Verpuffungen führen können und damit den sicheren Betrieb des Reaktors gefährden, werden ebenfalls nicht beobachtet.

Die Herstellung von Carbonsäuren und Carbonsäureanhydriden, insbesondere Phthalsäureanhydrid durch katalytische Gasphasenoxidation von aromatischen Kohlenwasserstoffen, insbesondere o-Xylol unter Verwendung der erfindungsgemäß hergestellten Katalysatoren erfolgt in an sich bekannter Weise, wie dies z.B. in K. Towae, W. Enke, R. Jäckh, N. Bhargawa "Phthalic Acid and Derivatives" in Ullmann's Encyclopedia of Industrial Chemistry Vol. A 20, 1992, S. 181 zusammenfassend dargestellt ist. Dabei werden vorzugsweise 2 oder mehr Katalysatorschichten angewandt, von denen vorzugsweise nur eine Schicht gaseintrittsseitig mit den erfingungsgemäßen Katalysatoren beschickt wird, da gasaustrittsseitig die Katalysatorbelastung geringer ist und deshalb ein Standardkatalysator ausreicht. Im einzelnen geht man z.B. beim Einsatz der neuen Katalysatoren so vor, daß zunächst die Katalysatoren in die Reaktionsrohre des Reaktors, die von außen auf die Reaktionstemperatur, beispielsweise mittels Salzschmelzen, thermostatisiert sind, gefüllt wird. Über die so bereitete Katalysatorschüttung wird das Reaktionsgas bei Temperaturen von im allgemeinen 300 bis 450°C, vorzugsweise 320 bis 420°C und besonders bevorzugt von 340 bis 400°C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h⁻¹ geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das molekularen Sauerstoff enthaltende Gas im allgemeinen mit 30g bis 150g je Nm³ Gas des zu oxidierenden, aromatischen Kohlenwasserstoffs beschickt.

### Beispiel 1: Herstellung von Standardkatalysators I ohne Binderzusatz (Vergleichsbeispiel)

50,0 kg Steatit (Magnesiumsilikat) Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 25,0 kg Anatas (Analyse: 0,18 % S; 0,08 % P; 0,24 % Nb; 0,01 Na; 0,01 K; 0,004 % Zr; 0,004 % Pb) einer BET-Oberfläche von 20 m²/g, 1,81 kg Vanadyloxalat, 0,143 kg Cäsiumsulfat, 38 kg Wasser und 9,85 kg Formamid solange besprüht, bis das Gewicht der auf diese Weise auf getragenen Schicht 10,0 % des Gesamtgewichts (nach Calcination bei 450°C) des fertigen Schalenkatalysators betrug. Die auf diese weise aufgebrachte katalytisch aktive Masse, also die Katalysatorschale, bestand aus 0,40 Gew.-% Cäsium (berechnet als Cs), 4,0 Gew.-% Vanadium (berechnet als V₂O₅) und 95,6 Gew.-% Titandioxid. Zur Beschichtung wurden 17,8 kg Maische benötigt, d.h. ca. 12 % der versprühten Maische gingen bei Auftragen durch Austrag verloren. Der Abrieb nach dreifachem Falltest* betrug 14,4 %, nach Calcination bei 400°C betrug der Abrieb 23,7 %
* Falltest: Ca. 50 G Katalysator werden durch ein 3 m langes Rohr mit lichtem Durchmesser von 25 mm fallengelassen. Der Katalysator fällt in eine unter dem Rohr stehende Schale, wird von dem bei Aufschlag entstehenden Staub abgetrennt und wieder durch das Rohr fallengelassen. Der Gesamtmassenverlust nach dreimaligem Falltest bzgl. der aufgetragenen Aktivmassenmenge (= 100%) ist ein Maß für die Abriebfestigkeit des Katalysators. Beim Falltest nach Caicination wird wie oben verfahren, nachdem 50 g Katalysator 1 h auf 400°C erhitzt worden waren.

### Beispiel 2

a) Herstellung des Bindemittels (gemäß WO 97/31036)
Ein Copolymer aus Acrylsäure/Maleinsäure in Gewichtsverhältnis 75:25 wird bei 110°C mit Wasserstoffperoxid als Radikalinitiator entsprechend den Angaben der EP-A 75 820 polymerisiert. Der Feststoffgehalt der erhaltenen Polymerlösung beträgt 44,6 %, der pH-Wert 0,7 und das M_{w}: 90 000. Man mischt eine 882,0 g Polymer enthaltende Polymerlösung mit 118,0 g Triethanolamin. Da so erhaltene Bindemittel hat einen Feststoffgehalt von 49,4 Gew.-% eine pH-Wert von 2,9 und eine Viskosität von 3700 mPas.
b) Herstellung der erfindungsgemäßen Katalysatoren A-C mit Binderzusatz
Man verfährt wie unter Beispiel 1 angegeben mit der Maßgabe, daß jeweils 17,0 kg Maische vor dem Aufsprühen auf den Katalysaor mit 400g, 800g, bzw. 1050g des wäßrigen Bindemittels gemäß (a) versetzt wurden. Die Steatiringe wurden mit der so modifizierten Maische solange besprüht, bis das Gewicht der auf diese Weise aufgetragenen Schicht 10,0 % des Gesamtgewichts (nach Calcination bei 450°C) des fertigen Schalenkatalysator betrug. Notwendige Maischemengen, Maischeverlust und Abriebwerte sind in de nachfolgenden Tabelle 1 aufgelistet.

**Tabelle 1:**

| Katalysator | 49.4%ige Bindemittellösung | Maischeverbrauch | Maischeverlust | Bindemittelgehalt der Aktivmassen | Abrieb 1 | Abrieb 2 |
|---|---|---|---|---|---|---|
| A | 400g | 16,2kg | 2% | 3,6% | 8,2% | 11,4% |
| B | 800g | 16,5kg | 4% | 7,2% | 3,2% | 12,9% |
| C | 1050g | 16,3kg | 3% | 9,5% | 2,3% | 20,1% |
| Abrieb 1: Falltest mit Originalprobe; Abrieb 2: Falltest mit calcinierter Probe (400°C) | | | | | | |

### Abrennen des Binderzusatzes

Zur Überprüfung, ob beim Abbrand des Binderzusatzes geruchsbelästigende bzw. umweltbeeinträchtigende Stoffe freigesetzt werden, wurde Katalysator C unter Durchleitung von Luft von 30°C auf 610°c (Temperatursteigerung: 5°C/min) erhitzt; die Massenabnahme sowie die entstehenden gasförmigen (Zersetzungs-)Produkte wurden on-line mit Hilfe der gekoppelten Differentiellen-Thermogravimetrie/FTIR-Spektroskopie analysiert. Es wurde festgestellt, daß alle flüchtigen Bestandteile bis 400°C entfernt werden; des weiteren sind in der Gasphase nur H₂O, CO und CO₂ nachweisbar. Geruchsbelästigende, umweltbeeinträchtigende bzw. entzündliche Zersetzungsprodukte konnten nicht identifiziert werden.

### Beispiel 3: Herstellung eines Standardkatalysaors II ohne Binderzusatz

50 kg Steatit (Magnesiumsilikat) Ringe mit einem äußeren Durchmesser von 8 mm, einer Länge von 6 mm und einer Wandstärke von 1,5 mm wurden in einer Dragiertrommel auf 160°c erhitzt und mit einer Suspension aus 28,6 kg Anatas mit einer BET-Oberfläche von 20 m²/g, 4,11 kg Vanadyloxalat, 1,03 kg Antimontrioxid, 0,179 kg Ammoniumhydrogenphosphat, 0,046 kg Cäsiumsulfat, 44,1 kg Wasser und 9,14 kg Formamid besprüht, bis das Gewicht der aufgetragenen Schicht 10,5 % des Gesamtgewichts des fertigen Katalysators betrug (nach Calcination bei 450°c). Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als V₂O₅), 3,3 Gew.-% Antimon (berechnet al Sb₂O₃), 0,1 Gew.-% Cäsium (berechnet als Cs) und 89,05 Gew.-% Titandioxid. Zur Beschichtung wurden 17,2 kg Maische benötigt, d.h. ca. 6 % der versprühten Maische gingen beim Auftragen durch Austrag verloren. Der Abrieb nach dreifachem Falltest betrug 8,0 %.

### Beispiel 4: Herstellung des erfindungsgemäßen Katalysators D und eines Vergleichskatalysators E mit über 10.-% Binderzusatz

Man verfährt wie in Beispiel 3 angegeben, mit der Maßgabe daß jeweils 17,0 kg Maische vor dem Aufsprühen auf den Katalysator mit 700 g bzw. 1500 g der wäßrigen Bindemittel gemäß Beispiel 2a, versetzt wurde. Die Steatiringe wurden mit de so modifizierten Maische solange besprüht, bis das Gewicht der auf diese Weise aufgetragenen Schicht 10,5 % des Gesamtgewichts (nach Calcination bei 450°C) des fertigen Schalenkatalysators betrug. Notwendige Maischemengen, Maischeverlust und Abriebwerte sind in nachfolgender Tabelle 2 aufgelistet.

**Tabelle 2:**

| Katalysator | Bindemittellösung | Maischeverbrauch | Maischeverlust | Bindemittelgehalt der Aktivmasse | Abrieb 1 | Abrieb 2 |
|---|---|---|---|---|---|---|
| D | 700g | 16,4kg | 2% | 6,3% | 5,2% | 19,6% |
| E | 1500g | 16,3kg | 1% | 13,4% | 3,3% | 51,3% |
| Abrieb 1: Falltest mit Originalprobe; Abrieb 2: Falltest mit calcinierter Probe (400°C) | | | | | | |

### Beispiel 5: Herstellung von Phthalsäureanhydrid

Von unten nach oben wurden jeweils 1,30 m des Katalysators II und anschließend 1,60 m der Katalysatoren I (Vergleich) bzw. C (erfinderisch) in ein 3,85 m langes Eisenrohr mit einer lichten Weite von 25mm eingefüllt. Das Eisenrohr war zur Temperaturregelung von einer Salzschmelze umgeben. Durch das Rohr wurden stündlich von oben nach unten 4,0Nm³-Luft mit Beladungen an 98,5 Gew.-%igem o-Xylol von 40 bis etwa 80g/Nm³-Luft geleitet. Dabei wurden bei 75-85 g Beladung die in folgender Tabelle 3 zusammengefaßten Ergebnisse erhalten (Ausbeute bedeutet das erhaltene Phtalsäureanhydrid (PSA) in Gewichtsprozent, bezogen auf 100%iges o-Xylol; Hochfahrzeit bedeutet die zur Beladungserhöhung von 40 auf 80g/Nm³ benötigten Tage).

**Tabelle 3:**

| Beispiel Katalysatorkombination | Salzbadtemperatur (°C) | durchschnittliche PSA-Ausbeute (gew.-%) | Hochfahrzeit (d) |
|---|---|---|---|
| I/II (Vergleich) | 352 | 113,3 | 36 |
| C/II | 354 | 113,5 | 25 |

### Beispiel 6 - 15

Im wesentlichen gleiche Ergebnisse bezüglich Abrieb 1, Abrieb 2, PSA-Ausbeute und Hochfahrzeit werden erhalten, wenn man zur Katalysatorherstellung die Bindemittel der folgende Tabelle 4 verwendet.

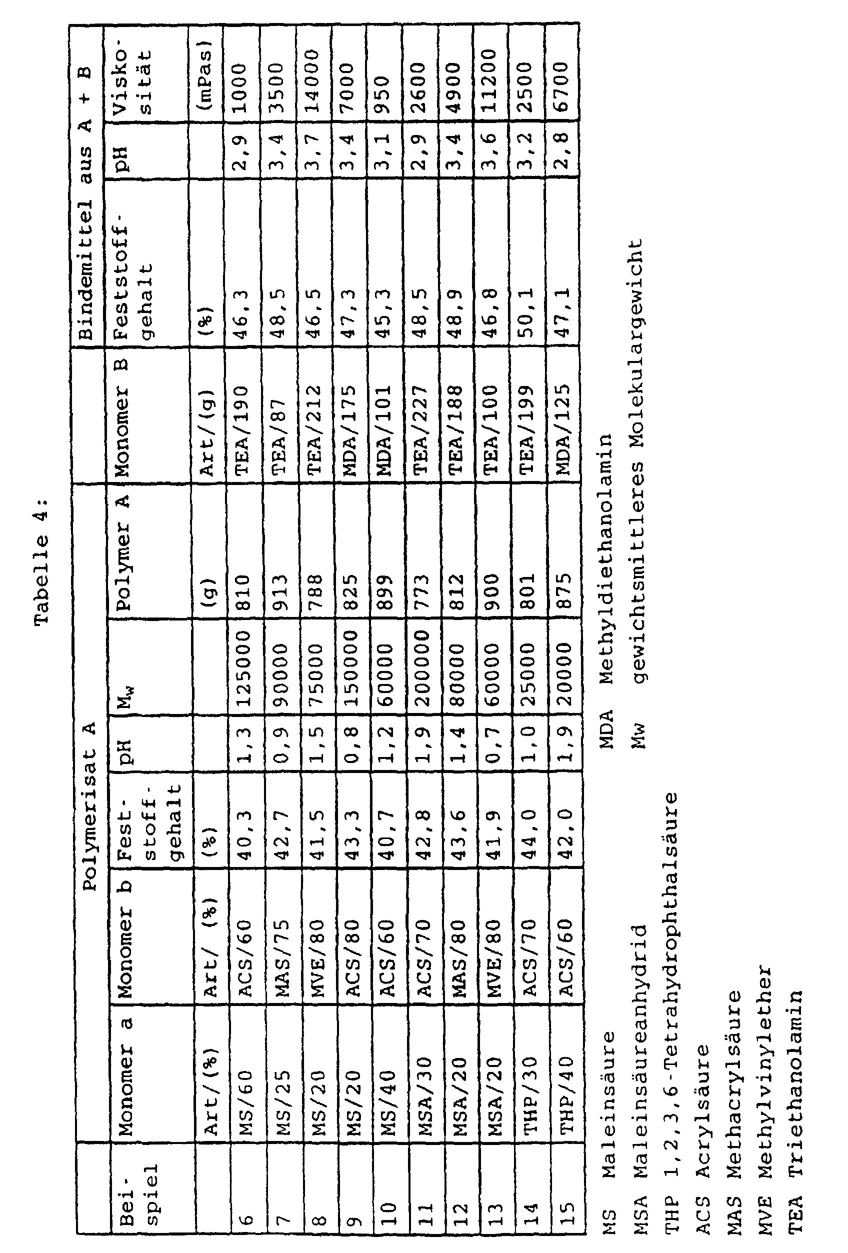

## Patentansprüche

1. Verfahren zur Herstellung von Schalenkatalysatoren für die katalytische Gasphasenoxidation von aromatiechen Kohlenstoffen zu Carbonsäuren und/oder Carbonsäureanhydriden, bestehend aus einem Trägerkern und darauf schalenförmig aufgebrachten katalytisch wirksamen Metalloxiden, erhalten durch Aufsprühen einer die aktiven Metalloxide enthaltenden wässrigen Aktivmassensuspension bei höheren Temperaturen auf das 50 bis 450°C heiße Trägermaterial, **dadurch gekennzeichnet, daß** die wässrige Aktivmassensuspension 1 bis 10 Gew.-%, bezogen auf den Feststoffgehalt der Aktivmassensuspension, eines Bindemittels enthält, bestehend aus
A) einem durch radikalische Polymerisation erhaltenen Polymerisat, enthaltend 5 bis 100 Gew.-% Monomere (a) in Form von ethylenisch ungesättigten Säureanhydriden oder ethylenisch ungesättigten Dicarbonsäuren, deren Carbonylgruppen ein Anhydrid bilden können und 0 bis 95 Gew.-% weiterer monoethylenisch ungesättigter Monomere (b), mit der Maßgabe, daß die Monomeren (a) und (b) durchschnittlich höchstens 5 Kohlenstoffatome, die nicht durch Sauerstoff enthaltende Gruppen funktionaliaiert sind, aufweisen und
B) einem Alkanolamin mit mindestens 2 OH-Gruppen, höchetens 2 Stickstoffatomen und höchstens 8 C-Atomen,
wobei das Gewichtsverhältnis A:B 1:0, 05 bis 1:1 beträgt.

2. Verfahren nach Anspruch 1, **dadurch gekennzeichnet, daß** die Monomeren (a) und (b) 2 bis 4 C-Atome und die Alkanolamine ein Stickstoffatom aufweisen.

3. Verfahren nach Anspruch 1, **dadurch gekennzeichnet, daß** im Bindemittel aus (A) und (B) das Atomverhältnis C:O höchstens 3:1 beträgt.

4. Verfahren nach Anspruch 1, **dadurch gekennzeichnet, daß** die Temperatur des zu beschichtenden Trägermaterials 100-250°C beträgt.

5. Verfahren nach Anspruch 1, **dadurch gekennzeichnet, daß** die Temperatur des zu beschichtenden Trägermaterials 140-200°C beträgt.

6. Verfahren nach den Ansprüchen 1 bis 3, **dadurch gekennzeichnet, daß** die organischen Bestandteile des Binderzusatzes 4-8 Gew.-%, bezogen auf den Festatoffgehalt der Aktivmassen-Suspension, betragen.

7. Schalkatalysatoren für die katalytische Gasphasenoxidation von aromatischen Kohlenwasserstoffen zu Carbonsäuren und/oder Carbonsäureanhydriden, auf deren Trägermaterial aus Quarz, Porzellan, Magnesiumoxid, Siliciumcarbid, Zinnoxid, Rutil, Tonerde, Aluminiumsilikat, Magnesiumsilikat, Zirkoniumsilikat und/oder Cersilikat eine Schicht katalytisch aktiver Metalloxide schalenförmig aufgebracht ist, welche neben Titandioxid des Anatastyps und Vanadiumpentoxid Oxide der Alkalimetalle, der Erdalkalimetalle, des Thalliums, Aluminiums, Zirkoniums, Eisens, Nickels, Kobalts, Mangans, Zinns, Silbers, kupfers, Chroms, Molybdäns, Wolframs, Iridiums, Tantals, Niobs, Arsens, Antimons, Cers und/oder des Phosphors enthält, **dadurch gekennzeichnet, daß** der Schalenkatalysator nach dem Verfahren gemäß Ansprach 1 herstellbar ist.

8. Schalenkatalysator für die katalytische Gasphasenoxidaton von aromatischen Kohlenwasserstoffen zu Carbonsäuren oder Carbonsäureanhydriden, bestehend aus einem im wesentlichen inerten Trägerkern aus Quarz, Porzellan, Magnesiumoxid, Siliziumcarbid, zinnoxid, Rutil, Tonerde, Aluminiumsilikat, Magnesiumsilikat, Zirkonsilikat und/oder Cersilikat und einer in Mengen von 5 bis 20 Gew.-%, bezogen auf die Menge des Trägers, auf dem Trägerkern aufgebrachten schalenförmigen Schicht katalytisch aktiver Metalloxide enthaltend als wesentliche Bestandteile Titanoxid vom Anatastyp und Vanadinpentoxid sowie ein Bindemittel, **dadurch gekennzeichnet, daß** das Bindemittel im wesentlichen aus
A) einem durch radikalische Polymerisation erhaltenen Polymerisat, enthaltend 5 bis 100 Gew.-% Monomere (a) in Form von ethylenisoh ungesättigten Säureanhydriden oder ethylenisch ungesättigten Dicarbonsäuren, deren Carbonylgruppen ein Anhydrid bilden können und 0 bis 95 Gew.-% weiterer monoethylenisch ungesättigter Monomere (b), mit der Maßgabe, daß die Monomeren (a) und (b) durchschnittlich höchstens 6 Kohlenatoffatome aufweisen und
B) einem Alkanolamin mit mindestens 2 OH-Gruppen, höchstens 2 Stickstoffatomen und höchstens 6 C-Atomen besteht und daß der Gehalt des Bindemittels, bezogen auf die Menge der aktiven Metalloxide 1 bis 10 Gew.-% beträgt,
wobei das Gewichtsverhältnis A:B 1:0,05 bis 1:1 beträgt.

9. Verwendung von Katalysatoren gemäß Anspruch 8 für die katalytische Gasphasenoxidation von aromatischen Kohlenwässerstoffen zu Carbonsäuren und/oder Carbonsäureanhydriden mit einem molekularen Sauerstoff enthaltenden Gas in einem Festbett bei erhöhter Temperatur.

10. Verwendung von Katalysatoren gemäß Anspruch 8 für die katalytische Gasphasenoxidation von Xylol und/oder Naphthalin zu Phthalsäureanhydrid mit einem molekularen Sauerstoff enthaltenden Gas in einem Festbett bei erhöhter Temperatur.

11. Verfahren zur katalytischen Gasphasenoxidation von aromatischen Kohlenwasserstoffen zu Carbonsäuren und/oder Carbonsäureanhydriden mit einem molekularen Sauerstoff enthaltenden Gas in einem Festbett bei erhöhter Temperatur und mittels einem oder mehreren in Schichten im Reaktor angeordneten Schalenkatalysatoren auf deren Trägermaterial eine Schicht aus katalytisch aktiven Metalloxiden schalenförmig aufgebracht ist, **dadurch gekennzeichnet, daß** man mindestens einen Schalenkatalysator gemäß Anspruch 8 verwendet.

## Claims

1. A process for the preparation of coated catalysts for the catalytic gas-phase oxidation of aromatic hydrocarbons to carboxylic acids and/or carboxylic anhydrides, consisting of a carrier core and catalytically active metal oxides applied thereto in shell form, obtained by spraying an aqueous active material suspension containing the active metal oxides at relatively high temperatures onto the carrier material at 50 to 450°C, wherein the aqueous active material suspension contains 1 to 10% by weight, based on the solids content of the active material suspension, of a binder, consisting of
A) a polymer obtained by free-radical polymerization, comprising 5 to 100% by weight of monomers (a) in the form of ethylenically unsaturated acid anhydrides or ethylenically unsaturated dicarboxylic acids whose carbonyl groups can form an anhydride and 0 to 95% by weight of further monoethylenically unsaturated monomers (b), with the proviso that the monomers (a) and (b) on average have at most 5 carbon atoms which are not functionalized by groups comprising oxygen, and
B) an alkanolamine having at least 2 OH groups, at most 2 nitrogen atoms and at most 8 C atoms,
the weight ratio A:B being 1:0.05 to 1:1.

2. A process as claimed in claim 1, wherein the monomers (a) and (b) have 2 to 4 C atoms and the alkanolamines have a nitrogen atom.

3. A process as claimed in claim 1, wherein, in the binder formed from (A) and (B), the atomic ratio C:O is at most 3:1.

4. A process as claimed in claim 1, wherein the temperature of the carrier material to be coated is 100-250°C.

5. A process as claimed in claim 1, wherein the temperature of the carrier material to be coated is 140-200°C.

6. A process as claimed in claims 1 to 3, wherein the organic constituents of the binder additive are 4-8% by weight, based on the solids content of the active material suspension.

7. A coated catalyst for the catalytic gas-phase oxidation of aromatic hydrocarbons to carboxylic acids and/or carboxylic anhydrides, to the support material comprising quartz, porcelain, magnesium oxide, silicon carbide, tin oxide, rutile, alumina, aluminum silicate, magnesium silicate, zirconium silicate and/or cerium silicate of which is applied a layer of catalytically active metal oxides in shell form, which in addition to titanium dioxide of the anatase type and vanadium pentoxide contains oxides of the alkali metals, the alkaline earth metals, of thallium, aluminum, zirconium, iron, nickel, cobalt, manganese, tin, silver, copper, chromium, molybdenum, tungsten, iridium, tantalum, niobium, arsenic, antimony, cerium and/or of phosphorus, wherein the coated catalyst can be prepared by the process as claimed in claim 1.

8. A coated catalyst for the catalytic gas-phase oxidation of aromatic hydrocarbons to carboxylic acids or carboxylic anhydrides, consisting of an essentially inert carrier core of quartz, porcelain, magnesium oxide, silicon carbide, tin oxide, rutile, alumina, aluminum silicate, magnesium silicate, zirconium silicate and/or cerium silicate and a layer of catalytically active metal oxides applied to the carrier core in amounts from 5 to 20% by weight, based on the amount of the carrier, in shell form comprising as essential constituents titanium oxide of anatase type and vanadium pentoxide as well as a binder, wherein the binder essentially consists of
A) a polymer obtained by free-radical polymerization, comprising 5 to 100% by weight of monomers (a) in the form of ethylenically unsaturated acid anhydrides or ethylenically unsaturated dicarboxylic acids whose carbonyl groups can form an anhydride and 0 to 95% by weight of further monoethylenically unsaturated monomers (b), with the proviso that the monomers (a) and (b) on average have at most 6 carbon atoms and
B) an alkanolamine having at least 2 OH groups, at most 2 nitrogen atoms and at most 6 C atoms and wherein the content of the binder, based on the amount of the active metal oxides, is 1 to 10% by weight,
the weight ratio A:B being 1:0.05 to 1:1.

9. The use of catalysts as claimed in claim 8 for the catalytic gas-phase oxidation of aromatic hydrocarbons to carboxylic acids and/or carboxylic anhydrides using a gas comprising molecular oxygen in a fixed bed at elevated temperature.

10. The use of catalysts according to claim 8 for the catalytic gas-phase oxidation of xylene and/or naphthalene to phthalic anhydride using a gas comprising molecular oxygen in a fixed bed at elevated temperature.

11. A process for the catalytic gas-phase oxidation of aromatic hydrocarbons to carboxylic acids and/or carboxylic anhydrides using a gas comprising molecular oxygen in a fixed bed at elevated temperature and by means of one or more coated catalysts arranged in the reactor in layers, to the carrier material of which is applied a layer of catalytically active metal oxides in shell form, which comprises using at least one coated catalyst as claimed in claim 8.

## Revendications

1. Procédé de fabrication de catalyseurs en gaine pour l'oxydation catalytique en phase gazeuse d'hydrocarbures aromatiques en des acides carboxyliques et/ou anhydrides carboxyliques, constitués d'un coeur support et d'oxydes métalliques à action catalytique, appliqués par-dessus à la manière d'une gaine, que l'on obtient par pulvérisation, à haute température, d'une suspension aqueuse de masse active contenant les oxydes métalliques actifs sur le matériau support chaud à une température de 50 à 450°C, **caractérisé en ce que** la suspension aqueuse de masse active contient de 1 à 10 % en poids, par rapport à l'extrait sec de la suspension de masse active, d'un liant constitué
A) d'un polymère obtenu par polymérisation radicalaire, contenant de 5 à 100 % en poids de monomères (a) sous forme d'anhydrides d'acide à insaturation éthylénique ou d'acides dicarboxyliques à insaturation éthylénique dont les groupes carbonyle peuvent former un anhydride, et de 0 à 95 % en poids d'autres monomères à insaturation monoéthylénique (b), à la condition que les monomères (a) et (b) contiennent en moyenne au plus 5 atomes de carbone, qui ne soient pas fonctionnalisés par des groupes contenant de l'oxygène, et
B) d'une alcanolamine ayant au moins deux groupes OH, au plus 2 atomes d'azote et au plus 8 atomes de carbone,
le rapport en poids A:B étant de 1:0,05 à 1:1.

2. Procédé selon la revendication 1, **caractérisé en ce que** les monomères (a) et (b) contiennent de 2 à 4 atomes de carbone, et les alcanolamines contiennent un atome d'azote.

3. Procédé selon la revendication 1, **caractérisé en ce que**, dans le liant constitué de (A) et de (B), le rapport en atomes C:O est d'au plus 3:1.

4. Procédé selon la revendication 1, **caractérisé en ce que** la température du matériau support à revêtir est de 100 à 250°C.

5. Procédé selon la revendication 1, **caractérisé en ce que** la température du matériau support à revêtir est de 140 à 200°C.

6. Procédé selon les revendications 1 à 3, **caractérisé en ce que** les constituants organiques de l'additif liant représentent de 4 à 8 % de l'extrait sec de la suspension de masse active.

7. Catalyseurs en gaine pour l'oxydation catalytique en phase gazeuse d'hydrocarbures aromatiques en acides carboxyliques et/ou anhydrides carboxyliques, sur le matériau support desquels, en quartz, porcelaine, oxyde de magnésium, carbure de silicium, oxyde d'étain, rutile, alumine, silicate d'aluminium, silicate de magnésium, silicate de zirconium et/ou silicate de cérium, on applique sous forme d'une gaine une couche d'oxydes métalliques à activité catalytique, qui, outre du dioxyde de titane de type anatase et du pentoxyde de vanadium, contient des oxydes des métaux alcalins, des métaux alcalino-terreux, du thallium, de l'aluminium, du zirconium, du fer, du nickel, du cobalt, du manganèse, de l'étain, de l'argent, du cuivre, du chrome, du molybdène, du tungstène, de l'iridium, du tantale, du niobium, de l'arsenic, de l'antimoine, du cérium et/ou du phosphore, **caractérisés en ce que** le catalyseur en gaine peut être préparé par le procédé selon la revendication 1.

8. Catalyseur en gaine pour l'oxydation catalytique en phase gazeuse d'hydrocarbures aromatiques en acides carboxyliques ou anhydrides carboxyliques, constitué d'un coeur support essentiellement inerte en quartz, porcelaine, oxyde de magnésium, carbure de silicium, oxyde d'étain, rutile, alumine, silicate d'aluminium, silicate de magnésium, silicate de zirconium et/ou silicate de cérium, et d'une couche d'oxydes métalliques à activité catalytique, en forme de gaine, appliquée sur le coeur support en des quantités de 5 à 20 % en poids par rapport à la quantité du support, et contenant en tant que constituants principaux de l'oxyde de titane de type anatase et du pentoxyde de vanadium, ainsi qu'un liant, **caractérisé en ce que** le liant est pour l'essentiel constitué
A) d'un polymère obtenu par polymérisation radicalaire, contenant de 5 à 100 % en poids de monomères (a) sous forme d'anhydrides d'acides à insaturation éthylénique ou d'acides carboxyliques à insaturation éthylénique, dont les groupes carbonyle peuvent former un anhydride, et de 0 à 95 % en poids d'autres monomères à insaturation monoéthylénique (b), à la condition que les monomères (a) et (b) aient en moyenne au plus 6 atomes de carbone, et
B) d'une alcanolamine ayant au moins deux groupes OH, au plus deux atomes d'azote et au plus 6 atomes de carbone, et **en ce que** la teneur en le liant, rapportée à la quantité des oxydes métalliques actifs, soit de 1 à 10 % en poids,
le rapport en poids A:B étant de 1:0,05 à 1:1.

9. Utilisation de catalyseurs selon la revendication 8 pour l'oxydation catalytique en phase gazeuse d'hydrocarbures aromatiques en acides carboxyliques et/ou anhydrides carboxyliques, avec un gaz contenant de l'oxygène moléculaire, en lit fixe à haute température.

10. Utilisation de catalyseurs selon la revendication 8 pour l'oxydation catalytique en phase gazeuse du xylène et/ou du naphtalène en anhydride phtalique, avec un gaz contenant de l'oxygène moléculaire, en lit fixe à haute température.

11. Procédé d'oxydation catalytique en phase gazeuse d'hydrocarbures aromatiques en acides carboxyliques et/ou anhydrides carboxyliques, avec un gaz contenant de l'oxygène moléculaire en lit fixe à haute température, et à l'aide d'un ou plusieurs catalyseurs en gaine, disposés en couches dans le réacteur, sur le matériau support desquels est appliquée en forme de gaine une couche d'oxydes métalliques à activité catalytique, **caractérisé en ce qu'**on utilise au moins un catalyseur en gaine selon la revendication 8.
